# EUROPEAN PATENT APPLICATION

(11) **EP 4 643 939 A1**
(43) Date of publication of application: **05.11.2025**
(21) Application number: 23912828.3
(22) Date of filing: 22.12.2023
(51) Int. Cl.: A61N 1/40, A61N 1/36, A61N 1/32, A61N 1/06, A61H 9/00

(54) **METHOD FOR CONTROLLING COSMETIC PROCEDURE APPARATUS**

(30) Priority: 29.12.2022 KR 20220188276; 29.12.2022 KR 20230131831
(71) Applicant: APR CO., LTD., Seoul 05551 (KR)
(72) Inventor: JI, Jong Chul, Seoul 08592 (KR); LEE, Gyoun Jung, Seoul 08592 (KR); PARK, Seung Woo, Seoul 08592 (KR)
(74) Representative: Herrmann, Daniel
(86) International application number: PCT/KR2023/021467
(87) International publication number: WO 2024/144153

(57) **Abstract**

A cosmetic procedure device control method is disclosed. The cosmetic procedure device control method according to one aspect of the present invention comprises: a skin suction step of forming negative pressure in a suction space so as to suction the skin into the suction space; a skin height detection step of detecting the height of the skin suctioned into the suction space; and a high frequency application determination step of determining whether to operate a high frequency application unit according to the height of the skin determined in the skin height detection step, wherein the skin height detection step detects which electrode end is in contact with the skin and conducts electricity from among a plurality of electrode ends of the high frequency application unit.

## Description

### TECHNICAL FIELD

The present invention relates to a method for controlling a cosmetic procedure device, and more specifically to a method for controlling a cosmetic procedure device that can perform a procedure more safely by measuring the height of the suctioned skin when suctioning the skin.

### BACKGROUND ART

In recent years, the spread of home cosmetic procedure devices that allow users to perform cosmetic procedures such as hair removal and skin care at home has been increasing.

Such home cosmetic procedure devices allow users to conveniently perform cosmetic procedures at their own home at any time they want, thereby lowering barriers to cosmetic procedures and increasing accessibility.

Meanwhile, as the spread of home cosmetic procedure devices increases, the performance of home cosmetic procedure devices is improving dramatically compared to the past, and the effectiveness of the procedures is also increasing.

For example, cosmetic procedure devices are being developed that can perform hair removal and massage functions by applying lasers, high frequency and the like, or perform a suction function that can intensively stimulate raised areas by suctioning the user's skin.

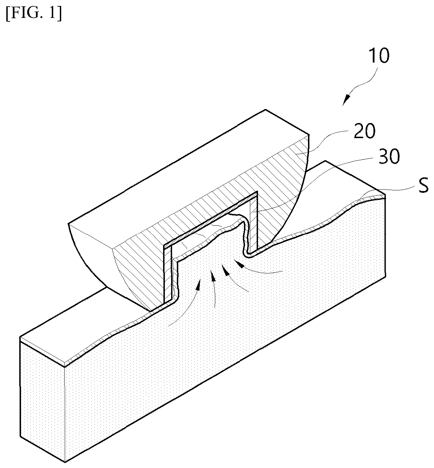

FIG. 1 is a diagram illustrating a conventional cosmetic procedure device 10 suctioning the skin S and applying high frequency to raised areas.

The conventional cosmetic procedure device 10 suctions the skin S into a header part 20 by using a suction force generated by a vacuum pump, and when the suctioned skin S comes into contact with an electrode, only a specific area can be treated with deep diathermy by applying high frequency to the contacted skin S without stimulating the sensory nerves and motor nerves, and the fat decomposition effect of the fat layer of the skin can be obtained through the thermal effect.

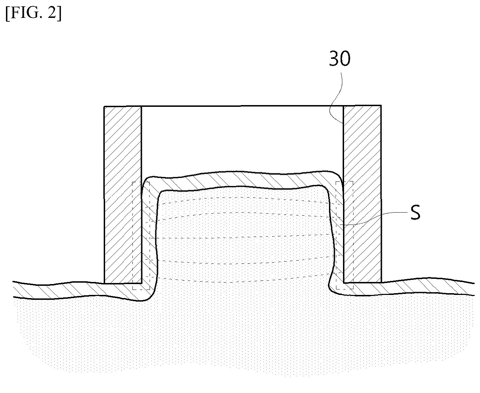

That is, as shown in FIG. 2, a high frequency electrode 30 is disposed inside the header part 20, and the skin S suctioned into the header part 20 contacts the high frequency electrode 30 to conduct electricity, and the procedure is performed by applying high-frequency energy to the skin S that is in contact with the electrode.

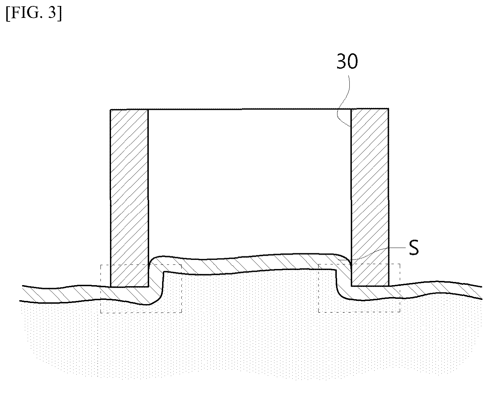

However, due to various reasons such as insufficient suction pressure or poor adhesion to the skin S, as shown in FIG. 3, if the amount of skin S suctioned into the header part 20 is not sufficient, the area of skin S that is in contact with a high frequency electrode 30 may be insufficient, and if high-frequency energy is applied in this state, a large amount of high-frequency energy is concentrated in a narrow contact area, and thus, there is a risk of causing a burn to the skin S.

In the case of Patent Document 1 (Korean Registered Patent Publication No. 10-2034272, the invention relates to a high-frequency output control device of a high-frequency stimulation treatment device and a high-frequency output control method using the same.

In Patent Document 1, in order to apply a high-frequency electric signal, the continuity of a current flow is checked from a current signal flowing through the skin, and by controlling the output intensity of high frequency waves that are output through the handpiece depending on the continuity of the current flow, the operator can easily control the output intensity of high frequency waves that are output from a high-frequency transmission device by using the high-frequency transmission device during the procedure, thereby increasing the convenience of operation during the procedure.

In other words, it is equipped to control a headpiece, such as adjusting the output intensity of high frequencies by allowing the user to remove the handpiece from the skin.

However, there has been no awareness of the problems that burns may occur when the height of the suctioned skin S is low and the contact area between the electrode applying high frequency and the skin is narrow.

### DISCLOSURE

### TECHNICAL PROBLEM

The present invention has been devised to solve the above problems, and an object of the present invention is to provide a cosmetic procedure device that ensures safety even when the contact area between the electrode and the skin is narrow due to a low height of the suctioned skin when the cosmetic procedure device is used.

The problems of the present invention are not limited to the problems mentioned above, and other problems that are not mentioned will be clearly understood by those skilled in the art from the description below.

### TECHNICAL SOLUTION

According to an aspect of the present invention, provided is a method for controlling a cosmetic procedure device, including a skin suction step of forming a negative pressure in a suction space so as to suction skin into the suction space; a skin height detection step of detecting a height of the skin suctioned into the suction space; and a high frequency application determination step of determining whether to operate a high frequency application unit according to the height of the skin determined in the skin height detection step, wherein the skin height detection step detects which electrode end is in contact with the skin and conducts electricity from among a plurality of electrode ends of the high frequency application unit.

The high frequency application determination step may operate the high frequency application unit if the height of the skin detected in the skin height detection step is higher than a preset height, and may not operate the high frequency application unit if the height of the skin determined in the skin height determination step is lower than a preset height.

### ADVANTAGEOUS EFFECTS

According to the above configuration, according to the method for controlling a cosmetic procedure device according to the present invention, the area of the skin that is in contact with the electrode is detected to determine whether the high frequency application unit operates, and thus, when the contact area between the electrode and the skin is narrow, the high frequency application unit does not operate. Therefore, even if an unskilled person uses it at home, there is less risk of side effects such as burns, which can improve user safety as well as product reliability.

In addition, even during use of the product, when the contact area between the electrode and the skin is reduced due to a user's operating error or device failure, the high frequency application unit does not operate, thereby improving user safety.

In addition, since the high frequency application unit operates when the contact area between the electrode and the skin is above an appropriate level, the user's safety is improved, and the treatment effect can also be maximized, thereby improving the user's satisfaction.

The effects of the present invention are not limited to the effects described above, and should be understood to include all effects that can be inferred from the configuration of the invention described in the detailed description or claims of the present invention.

### DESCRIPTION OF DRAWINGS

FIG. 1 is a diagram illustrating the suctioning of skin into a header part of a conventional cosmetic procedure device.
FIG. 2 is a diagram illustrating the application of high frequency after suctioning the skin to a header part in a conventional cosmetic procedure device.
FIG. 3 is a diagram illustrating a state in which the contact area between the electrode and the skin is insufficient in a conventional cosmetic procedure device.
FIG. 4 is a diagram illustrating a cosmetic procedure device according to an embodiment of the present invention.
FIG. 5 is a diagram illustrating the bottom surface of a cosmetic procedure device according to an embodiment of the present invention.
FIG. 6 is a diagram illustrating a high frequency application unit of a cosmetic procedure device according to an embodiment of the present invention.
FIG. 7 is a diagram illustrating a header part of a cosmetic procedure device according to an embodiment of the present invention.
FIG. 8 is a diagram illustrating a high frequency application unit of a cosmetic procedure device according to another embodiment of the present invention.
FIG. 9 is a diagram illustrating a header part of a cosmetic procedure device according to another embodiment of the present invention.
FIG. 10 is a flowchart showing an embodiment of the method for controlling a cosmetic procedure device according to the present invention.

### MODES OF THE INVENTION

Hereinafter, with reference to the attached drawings, embodiments of the present invention will be described in detail so that those skilled in the art can easily practice the present invention. The present invention may be implemented in many different forms and is not limited to the embodiments described herein. In order to clearly explain the present invention, parts that are not related to the description have been omitted in the drawings, and identical or similar components are assigned the same reference numerals throughout the specification.

The words and terms used in the present specification and claims are not to be construed as limited in their usual or dictionary meanings, but according to the principle that the inventor can define terms and concepts in order to explain his or her invention in the best way, they must be interpreted with meaning and concepts consistent with technical ideas.

Therefore, the embodiments described in the present specification and the configurations illustrated in the drawings correspond to preferred embodiments of the present invention and do not represent all of the technical ideas of the present invention, and therefore, the configurations may have various equivalents and modified examples that can replace the same at the time of filing of the present invention.

In the present specification, it should be understood that terms such as "include" or "have" are intended to describe the presence of a feature, number, step, operation, component, part or combination thereof described in the specification, but do not exclude in advance the possibility of the presence or addition of one or more other features, numbers, steps, operations, components, parts or combinations thereof.

When a component is said to be "in front of," "behind," "above" or "below" another component, unless there are special circumstances, it includes not only being positioned "in front of," "behind," "above" or "below" the other component in direct contact with the same, but also cases where there is another component disposed therebetween. Furthermore, when a component is said to be "connected" to another component, unless there are special circumstances, it includes cases where they are indirectly connected to each other as well as cases where they are directly connected to each other.

Hereinafter, a cosmetic procedure device according to an embodiment of the present invention will be described with reference to the drawings.

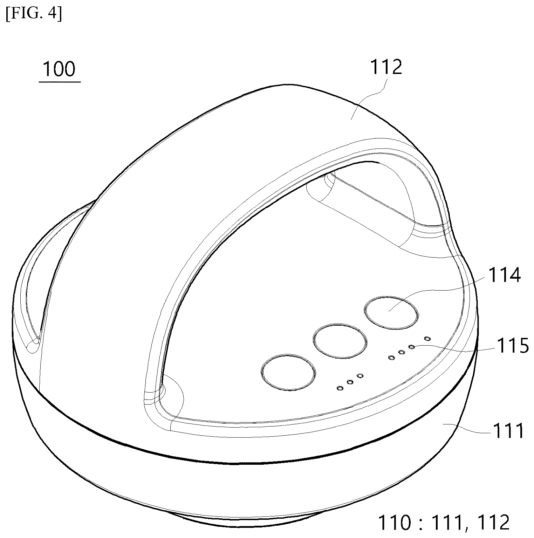

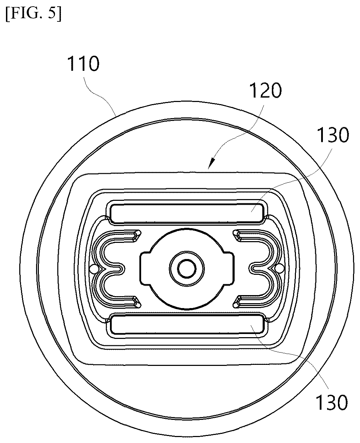

FIG. 4 is a diagram illustrating a cosmetic procedure device according to an embodiment of the present invention, and FIG. 5 is a diagram illustrating the bottom of a cosmetic procedure device according to an embodiment of the present invention.

The cosmetic procedure device according to an embodiment of the present invention performs a cosmetic procedure on the user's skin. More specifically, the cosmetic procedure device according to an embodiment of the present invention allows the user to perform a cosmetic procedure on his or her own skin. That is, the user can perform a cosmetic procedure on his or her own skin by contacting the cosmetic procedure device according to an embodiment of the present invention. For example, the cosmetic procedure device according to an embodiment of the present invention may apply high frequency to the skin of the user, which provides an anti-aging effect to the skin.

Referring to FIGS. 4 and 5, the cosmetic procedure device 100 according to an embodiment of the present invention includes a main body 110, a header part 120, a high frequency application unit 130, a skin height measurement unit 140 and a controller 150.

The main body 110 has a shape that can be in contact with a predetermined area of the user's skin. Components for the cosmetic procedure may be arranged on the main body 110. In an embodiment of the present invention, the header part 120 that can be in contact with a predetermined area of the user's skin is provided on a lower side of the main body 110.

The header part 120 may apply high frequency to the skin. The high frequency applied by the header part 120 may stimulate the user's skin and supply energy to provide an anti-aging effect.

In an embodiment of the present invention, the main body 110 includes a first part 111 provided such that the lower side is in contact with the user's skin and components for a cosmetic procedure are arranged, and a second part 112 connected to an upper side of the first part 111 such that the user can grasp the same. In this case, the first part 111 may be formed in a cylindrical shape, and the second part 112 may have an arch shape.

For example, a component for applying high frequency may be arranged in the main body 110. In addition, a pump 113 for providing a suction function that suctions in and stimulates the user's skin may also be arranged in the main body 110.

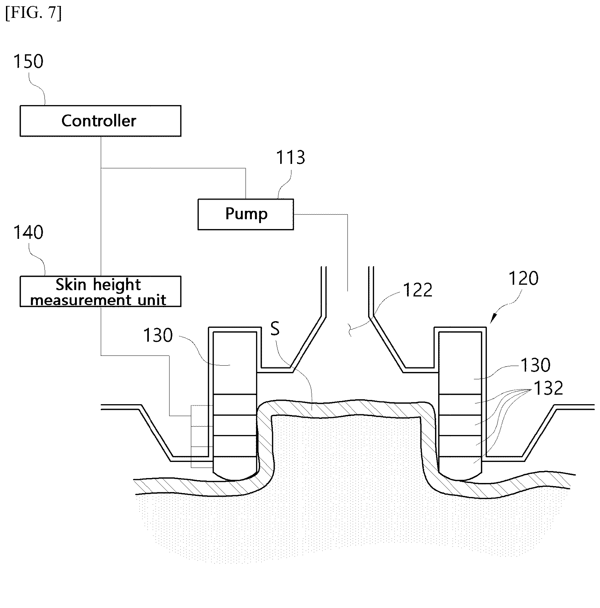

As shown in FIG. 7, a negative pressure is generated in the suction space 122 of the header part 120 formed at a lower open end of the main body 110 by the suction force generated by the pump 113, and accordingly, the user's skin is also suctioned into the suction space 122 while being raised. The pump 113 providing this suction function may be a pneumatic pump, but is not limited thereto. In other words, if it can generate a suction force, various structures and configurations may be applied.

Meanwhile, various technologies may be applied to the configuration and operation relationship for the suction function in the cosmetic procedure device 100, and a specific description thereof will be omitted in order to make the gist of the present invention clear.

That is, during the cosmetic procedure process, a lower part of the first part 111 comes into contact with the user's skin. In this regard, a suction space 122 in which the user's skin is suctioned may be formed in the lower part of the first part 111. The skin suctioned into the suction space 122 may receive high frequency through the high frequency application unit 130 arranged in the suction space 122.

Meanwhile, the second part 112 functions as a handle. The user may easily move the main body 110 while holding the second part 112, and may appropriately press the first part 111 toward the user's skin.

In addition, the second part 112 constituting an upper side of the main body 110 may be equipped with an operation unit 114 for receiving operation information for selecting on/off and various modes, and an output unit 115 for sending a warning signal through vibration and/or sound according to the control of the controller 150.

The header part 120 is arranged at a lower part of the first part 111 of the main body 110 and plays a role in performing a cosmetic procedure by directly contacting the user's skin.

Such a header part 120 may be manufactured in the form of an integral structure formed together with the first part 111 described above, or may be manufactured in the form of a detachable structure that is formed separately and can be detachably connected.

In the present embodiment, the header part 120 is expressed as a structure that constitutes a lower side of the first part 111 and is detachably connected.

The header part 120 is arranged in the lower side of the first part 111 of the main body 110 and directly contacts the user's skin S to perform a cosmetic procedure.

For example, such a suction space 122 may be in the form of a circle or an ellipse with a lower side open, and the cross-section thereof may be formed in the form of a polygon as needed.

However, as described above, such a suction space 122 causes the user's skin located within the suction space 122 to be uniformly raised upward by the negative pressure generated by the uniform suction force during the cosmetic procedure.

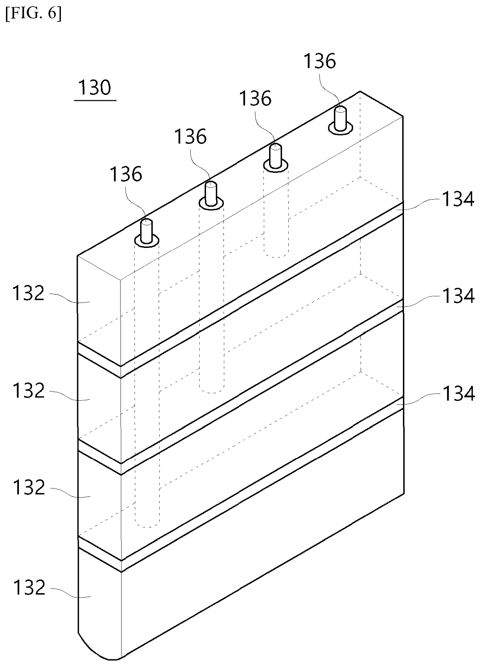

The high frequency application unit 130 is arranged on an inner surface of the header part 120 as shown in FIGS. 6 and 7, and is suctioned into the suction space 122 and comes into contact with the raised skin S, and at least one pair thereof may be provided to apply high frequency to the contacted skin S.

That is, as shown in FIG. 7, when the skin comes into contact with a pair of high frequency application units 130, current is generated, and thus, high frequency is applied while the skin is in a state of conducting electricity.

Meanwhile, the skin height measurement unit 140 may be provided to measure the height of the skin suctioned into the suction space 122 and raised.

In addition, the controller 150 may be provided to control the high frequency application unit 130 according to the height of the raised skin measured by the skin height measurement unit 140.

In this case, the skin height measurement unit 140 may be provided to detect a position where the skin comes into contact with the high frequency application unit 130 and conducts electricity.

Meanwhile, the high frequency application unit 130 may be arranged to face each other on both sides of the inner surface of the suction space 122 as shown in FIGS. 6 and 7, and may include a plurality of electrode ends 132 that are sequentially arranged in an upward direction, which is a direction in which the skin is suctioned into the suction space 122, and are electrically separated from each other.

That is, as shown in FIG. 6, the high frequency application unit 130 may be composed of a plurality of electrode ends 132 arranged in an upward direction, and an insulator 134 for electrical separation may be interposed between each electrode end 132.

In addition, a connection terminal 136 for electrical connection of each electrode end 132 may be arranged on the uppermost electrode end 132, and the connection terminal 136 of the electrode end 132 located on a lower side may extend upwardly by penetrating the electrode end 132 located on an upper side.

In addition, the skin height measurement unit 140 may be provided to detect which electrode end 132 is in contact with the skin S and conducts electricity from among a plurality of electrode ends 132 of the high frequency application unit 130. The skin height measurement unit 140 may be formed of an IC or a control circuit, and may be integrated into the controller 150 if necessary.

That is, the high frequency application unit 130 is provided with electrically separated electrode ends 132 arranged sequentially in the height direction, and the skin height measurement unit 140 detects which electrode end 132 is in contact with the skin S and conducts electricity from among the plurality of electrode ends 132.

Meanwhile, the controller 150 determines the height of the skin S suctioned and raised by the header part 120 as a position of the electrode end 132 that is in contact with the skin among the plurality of electrode ends 132 detected through the skin height measurement unit 140, and when the height of the skin S determined in this way is greater than or equal to a preset height, the controller 150 may control the application of high frequency to the skin S through each electrode end 132 of the high frequency application unit 130.

That is, as shown in FIG. 7, the skin S suctioned into the suction space 122 comes into contact with the high frequency application unit 130. In this case, since the high frequency application unit 130 is arranged in the height direction and is composed of a plurality of electrically separated electrode ends 132, if the height of the suctioned skin S is high, the electrode end 132 positioned at a high position will come into contact with the skin and conduct electricity. If the amount of suctioned skin S is small and the height of the suctioned skin is low, the skin will come into contact only with the electrode end 132 positioned at a low position.

Therefore, the controller 150 may determine the height of the skin S suctioned into the suction space 122 and raised, that is, the height of the skin S in contact with the high frequency application unit 130, and when the height of the determined skin S is higher than a preset height, it controls to apply high frequency to the skin through the high frequency application unit 130, and when the height of the determined skin S is lower than a preset height, it controls not to apply high frequency.

Therefore, when the amount of the skin suctioned into the suction space 122 and raised is small, that is, when the area of the skin in contact with the high frequency application unit 130 is small, high frequency is not applied, and only when the area of the skin S in contact with the high frequency application unit 130 is sufficient, it is possible to prevent the occurrence of side effects such as burns by blocking the energy applied by the high frequency application unit 130 from being concentrated on a small area of the skin S.

As described above, the controller 150 may determine the height of the skin as the position of the electrode end 132 that is in contact with the skin among the plurality of electrode ends 132, but may also determine the height of the skin S that is suctioned into the header part 120 and raised as the number of electrode ends 132 that are in contact with the skin from among the electrode ends 132 of the high frequency application unit 130.

That is, if the amount of skin S suctioned into the suction space 122 is large, the number of electrode ends 132 that are in contact with the skin S from among the electrode ends 132 of the high frequency application unit 130 may naturally increase, and thus, the height of the skin that is suctioned into the header part 120 and raised may be determined as the number of electrode ends 132 that conduct electricity from among the electrode ends 132 of the high frequency application unit 130.

Therefore, when the number of electrode ends 132 in contact with the skin is less than or equal to a set number, the high frequency is not applied, and when the number of electrode ends 132 in contact with the skin S is greater than or equal to a set number, the high frequency is applied, and thus, by blocking the energy applied from the high frequency application unit 130 from being concentrated on a narrow area of the skin S, it is possible to prevent side effects such as burns.

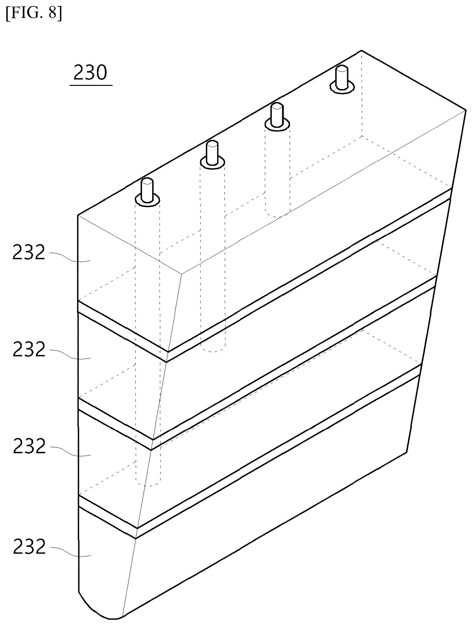

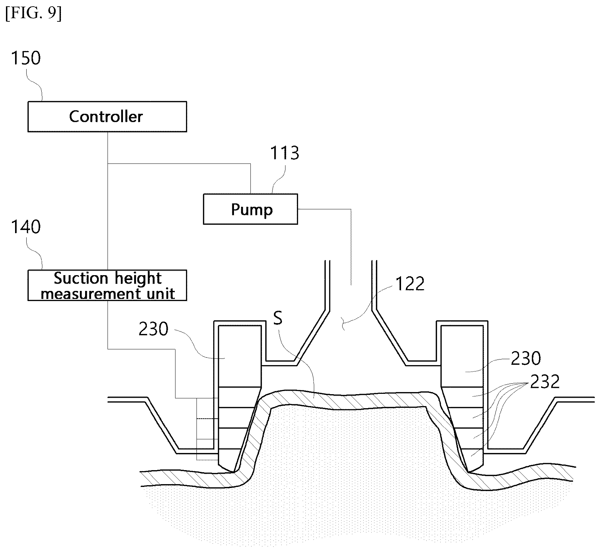

Meanwhile, as shown in FIGS. 8 and 9, the high frequency application unit 230 may be formed to be inclined so as to protrude inwardly from the header part 120 as it goes upward.

That is, among the plurality of electrode ends 232, the width of the electrode end 232 arranged at the lower side may be narrow, and the width of the electrode end 232 arranged at the upper side may be continuously inclined so as to be wide.

Certainly, it is not limited thereto, and the high frequency application unit 230 itself may be arranged to be inclined.

This is to ensure that the skin suctioned into the suction space 122 and raised, as shown in FIG. 9, adheres better to the high frequency application unit 230.

That is, as shown in FIG. 7, when the high frequency application unit 130 is arranged vertically, the skin suctioned into the suction space 122 must stretch in order to contact each electrode end 132 of the high frequency application unit 130. However, if the skin has insufficient elasticity, the suctioned skin may not make good contact with the high frequency application unit 130 even though it is sufficient.

Therefore, as shown in FIG. 9, when the high frequency application unit 230 is arranged such that it protrudes inwardly from the suction space 122 as it goes upward, there is an effect in which the skin raised in the suction space 122 may more easily contact each electrode end 232 of the high frequency application unit 230.

Hereinafter, an embodiment of the method for controlling a cosmetic procedure device that controls the cosmetic procedure device of the present invention will be described.

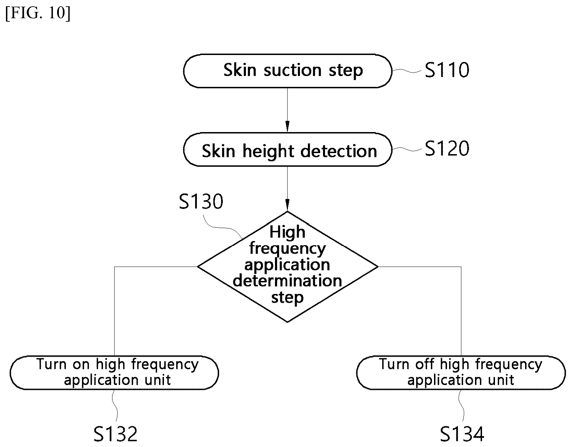

FIG. 10 is a flowchart showing an embodiment of the method for controlling a cosmetic procedure device according to the present invention.

The method for controlling a cosmetic procedure device according to the present embodiment may include a skin suction step S110, a skin height detection step S120 and a high frequency application determination step S130.

The skin suction step S110 is a step of operating a pump 113 to form a negative pressure in the suction space 122, and suctioning the skin S into the suction space 122 with the formed negative pressure.

In the skin suction step S110, the skin S suctioned into the suction space 122 may be raised as shown in FIG. 7 and FIG. 9 and may come into contact with the high frequency application unit 130 arranged on both inner side surfaces of the suction space 122.

The skin height detection step S120 is a step for detecting a height of the skin S suctioned into the suction space 122.

That is, the skin height measurement unit 140 detects which electrode end 132 of the high frequency application unit 130 composed of the plurality of electrode ends 132 is in contact with the skin and conducts electricity, and the controller 150 may determine the height of the skin S suctioned into the suction space 122 as the position information of the electrode end 132 that is in contact with the skin detected by the skin height measurement unit 140.

Alternatively, the skin height detection step S120 may also determine the height of the skin S suctioned into the suction space 122 as the number of electrode ends 132 that are in contact with the skin S and conduct electricity from among the plurality of electrode ends 132 of the high frequency application unit 130.

This is because, if the amount of skin S suctioned into the suction space 122 is large, the number of electrodes 132 in contact with the skin will also increase.

The high frequency application determination step S130 is a step in which, if the height of the skin S detected in the skin height detection step S120 is greater than or equal to a preset height, the high frequency application unit 130 is operated S132, and if the height of the skin determined in the skin height determination step is less than a preset height, the high frequency application unit 130 is not operated S134.

Therefore, the high frequency application unit 130 operates S132 only when the height of the skin suctioned into the suction space 122 is sufficient, and when the height of the suctioned skin is low, that is, when the amount of the suctioned skin is insufficient, the high frequency application unit 130 does not operate S134, and thus, it is possible to prevent the occurrence of burns due to high frequency energy being applied to a narrow area of skin.

In addition, this can be applied not only when the cosmetic procedure device 100 is first operated, but also when the height of the skin S suctioned into the suction space 122 of the header part 120 decreases during use, and thus, it is possible to further improve the user's safety.

Although the embodiments of the present invention have been described, the spirit of the present invention is not limited to the embodiments presented in the present specification, and those skilled in the art who understand the spirit of the present invention will be able to easily propose other embodiments by modifying, changing, deleting or adding components within the scope of the same spirit, but this will also be considered to fall within the spirit of the present invention.

## Claims

1. A method for controlling a cosmetic procedure device, the method comprising:
a skin suction step of forming a negative pressure in a suction space so as to suction skin into the suction space;
a skin height detection step of detecting a height of the skin suctioned into the suction space; and
a high frequency application determination step of determining whether to operate a high frequency application unit according to the height of the skin determined in the skin height detection step,
wherein the skin height detection step detects which electrode end is in contact with the skin and conducts electricity from among a plurality of electrode ends of the high frequency application unit.

2. The method of claim 1, wherein the high frequency application determination step operates the high frequency application unit if the height of the skin detected in the skin height detection step is higher than a preset height, and does not operate the high frequency application unit if the height of the skin determined in the skin height determination step is lower than a preset height.
